# EUROPÄISCHE PATENTANMELDUNG

(11) **EP 0 523 591 A2**
(43) Veröffentlichungstag der Anmeldung: **20.01.1993**
(21) Anmeldenummer: 92111893.1
(22) Anmeldetag: 13.07.1992
(51) Int. Cl.: A61K 35/78

(54) **Verwendung eines Kava-Extraktes und seiner Inhaltsstoffe**

(30) Priorität: 15.07.1991 DE 4123427
(71) Anmelder: Dr. Willmar Schwabe GmbH & Co., D-76209 Karlsruhe (DE)
(72) Erfinder: Krieglstein, Josef, Prof. Dr. Dr., W-3553 Coelbe (DE); Chatterjee, Shyam Sunder, Dr., W-7500 Karlsruhe 1 (DE); Stumpf, Heinz, Dr., W-7500 Karlsruhe 41 (DE)
(74) Vertreter: Bunke, Holger, Dr.rer.nat. Dipl.-Chem.

(57) **Zusammenfassung**

Es wird eine weitere medizinische Indikation für Kava-Extrakt und seine Inhaltsstoffe vorgeschlagen, nämlich die Verwendung als Neuroprotektivum, insbesondere bei der Behandlung und Prophylaxe von Hirnleistungsstörungen und Schlaganfällen.

## Beschreibung

Die Erfindung betrifft die therapeutische Verwendung eines Kava-Extraktes und seiner Inhaltsstoffe.

In Polynesien, der Heimat des Kava-Strauches (Piner methysticum Forst., Rauschpfeffer, Familie Piperaceae), verwenden die Eingeborenen seit jeher einen wäßrigen Auszug (Kaltmazerat) der Droge zu rituellen und therapeutischen Zwecken. Der aus dem Rhizom des Kava-Strauches gewonnene Extrakt wird "Kava-Kava" genannt.

Kava-Extrakt befindet sich im Handel und wird als Phytotranquilizer zur Entspannung bei Nervosität und Überreizung und als Mittel zur Schlafinduktion verwendet. Darüber hinaus wird in der Literatur über antikonvulsive, antiepileptische und spasmolytische Wirkungen des Kava-Extrakts und seiner Inhaltsstoffe berichtet (vgl. W. Kretschmer, Münchener Medizinische Wochenschrift Nr. 4/1970, Seite 154-158; R. Hänsel et al, Deutsche Apotheker Zeitung 125, Nr. 41, Seite 2056-2058 (1985); H. Krach, Zeitschrift für Allgemeinmedizin 62, S. 1028-1031 (1986)).

Soweit bisher bekannt, beruht die pharmakologische Wirksamkeit des Kava-Extraktes auf dem Vorkommen mehrerer 6-substituierter 4-Methoxypyrone, nämlich den Kavalactonen Kavain, Dihydrokavain, Methysticin, Dihydromethysticin, Yangonin und Desmethoxyyangonin.

Das Razemat von synthetisiertem Kavain ist in Form von Kapseln mit 200 mg des Wirkstoffs im Handel und wird als Psychopharmakon verwendet.

Es wurde nun gefunden, daß Kava-Extrakt und seine Inhaltsstoffe eine weitere medizinische Indikation besitzen: sie sind neuroprotektiv wirksam.

Der Erfindung liegt somit die Aufgabe zugrunde, eine weitere medizinische Indikation des Kava-Extrakts und seiner Inhaltsstoffe aufzufinden.

Diese Aufgabe wird erfindungsgemäß gelöst durch die Angabe der Verwendung des Extrakts oder seiner Inhaltsstoffe als Neuroprotektivum, insbesondere bei der Behandlung oder Prophylaxe von Hirnleistungsstörungen und Schlaganfällen, d.h. bei der Behandlung bzw. Prophylaxe von mit dem Extrakt und seinen Inhaltsstoffen in der Human- und Tiermedizin bisher noch nicht behandelten Krankheiten.

Unter "neuroprotektiver Wirksamkeit" wird die Fähigkeit verstanden, Nervenzellen des Gehirns vor Schädigungen unterschiedlichster Genese oder Abtötung zu schützen.

Für neue Arzneimittel mit neuroprotektiver Wirksamkeit besteht weltweit ein erhebliches Bedürfnis, da sie für die Therapie und Prophylaxe von Krankheiten geeignet sein können, die mit einem Untergang von Nervenzellen im Gehirn einhergehen. Dazu gehören Morbus Alzheimer, cerebro-vaskuläre Demenzen, Demenzen, die durch Infektionskrankheiten, Traumen des Gehirns, Chromosomenerkrankungen, Tumoren, Hydrocephalus, Stoffwechselkrankheiten, Mangelkrankheiten und Vergiftungen bedingt sein können. Gemeinsame Symptome dieser Erkrankungen sind Hirnleistungsstörungen, z.B. Störungen des Gedächtnisses, der Konzentrationsfähigkeit, des Denkens, der Auffassung, der Orientierung und der Affektivität.

Kava-Extrakt und seine Inhaltsstoffe können darüber hinaus in geeigneten Zubereitungsformen zur Therapie und Prophylaxe des Schlaganfalls (Apoplexia cerebri) sowie allgemein von Hirndurchblutungsstörungen verwendet werden.

Die neuroprotektive Wirksamkeit des Kava-Extrakts bzw. seiner Inhaltsstoffe wurde an Ratte und Maus untersucht. Der hierzu verwendete Kava-Extrakt enthielt 70 Gew.-% Kavalactone; er wurde durch Extraktion der Kava-Droge mit 70%igem Ethanol hergestellt (acetonlöslicher Teil). Als aus dem Extrakt isolierte Inhaltsstoffe wurden verwendet: (+)-Methysticin (Schmelzpunkt 130-133° C, aus Isopropanol) und (+)-Dihydromethysticin (Schmelzpunkt 113-114° C, aus Isopropanol). Den Versuchstieren wurde unter Narkose die mittlere Gehirnarterie dauerhaft durchtrennt und damit ein Gehirninfarkt bzw. fokale Ischämie erzeugt. Das Infarktvolumen bei der Ratte und die Infarktfläche an der Gehirnoberfläche der Maus wurden quantitativ bestimmt. Wie sich aus der Zeichnung ergibt, können Kava-Extrakt und seine Inhaltsstoffe das Ausmaß der Infarkte statistisch signifikant reduzieren. Eine solche Wirkung wird als neuroprotektiv oder cerebroprotektiv bezeichnet.
Fig. 1 ist eine grafische Darstellung der neuroprotektiven Wirkung des Kava-Extrakts, während
Fig. 2 eine grafische Darstellung der neuroprotektiven Wirkung der Kava-Extrakt-Inhaltsstoffe Methysticin und Dihydromethysticin ist.

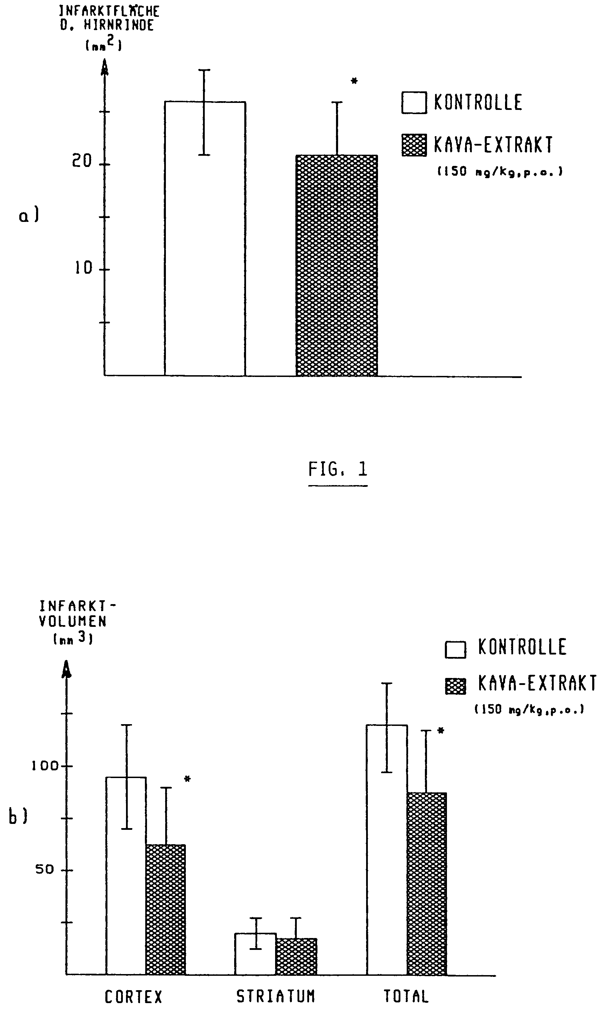

In Fig. 1a ist die Infarktfläche an der Oberfläche der Hirnrinde bei der Maus für unbehandelte Kontrolltiere (über 25 mm²) und für mit Kava-Extrakt behandelte Tiere (über 20 mm²) dargestellt.

In Fig. 1b ist das Infarktvolumen im Gehirn der Ratte für unbehandelte Kontrolltiere und für mit Kava-Extrakt behandelte Tiere dargestellt, und zwar jeweils für die Hirnrinde (Cortex), den Corpus striatum und die Summe (Total) aus diesen beiden Teilvolumina.

Die Infarktfläche bzw. das Infarktvolumen wurden 2 Tage nach Verschluß der Arteria cerebri media bestimmt. Der Kava-Extrakt wurde in einer Dosis von 150 mg/kg 1 Stunde vor dem Gefäßverschluß p.o. verabreicht. Angegeben sind Mittelwerte +/- Standardabweichung aus 9 bis 10 Versuchen. Der Kava-Extrakt reduzierte statistisch signifikant die Infarktfläche bzw. das Infarktvolumen: (*) p < 0.05, Mann-Whitney U-Test.

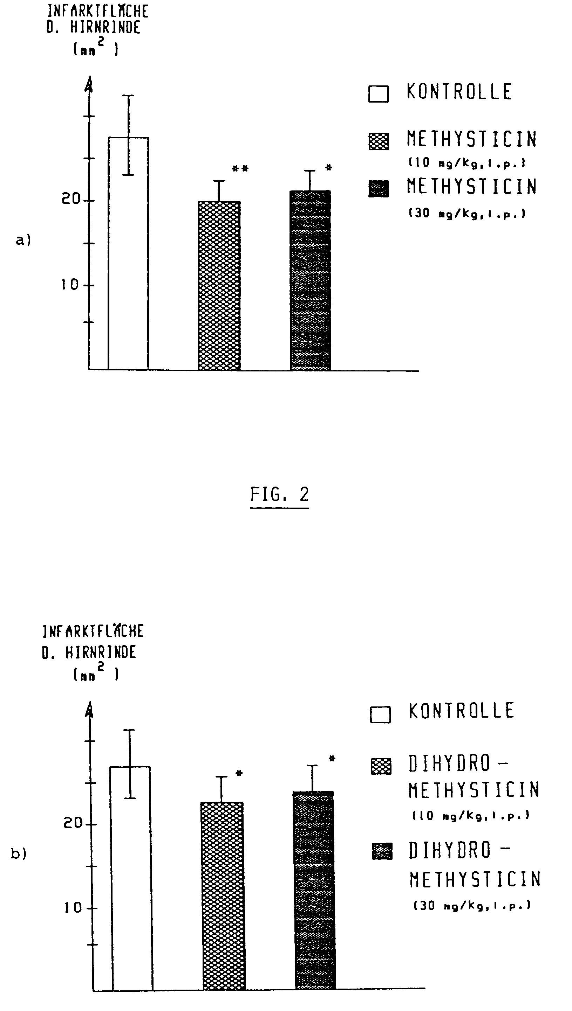

In gleicher Weise ist in Fig. 2a die Infarktfläche an der Gehirnoberfläche der Maus dargestellt für unbehandelte Tiere und für mit Methysticin in unterschiedlichen Dosen behandelte Tiere.

In Fig. 2b ist die Infarktfläche an der Gehirnoberfläche der Maus dargestellt für unbehandelte Tiere und für mit Dihydromethysticin in unterschiedlichen Dosen behandelte Tiere.

Auch hierbei wurde die Infarktfläche an der Gehirnoberfläche der Maus 2 Tage nach Verschluß der Arteria cerebri media gemessen. Methysticin und Dihydromethysticin wurden in Dosen von jeweils 10 bzw. 30 mg/kg 15 Minuten vor der Ischämie intraperitoneal injiziert. Angegeben sind Mittelwerte +/- Standardabweichung aus 9 bis 10 Versuchen. Beide Inhaltsstoffe des Kava-Extraktes reduzieren die Infarktfläche statistisch signifikant: (*) p < 0.05, (**) p < 0.01, Mann-Whitney U-Test.

Die neuroprotektive Wirksamkeit des Kava-Extrakts und seiner Inhaltsstoffe Methysticin und Dihydromethysticin wurde außerdem an kultivierten Neuronen von Hühnerembryonen nach der Methode von Ahlemeyer und Krieglstein (vgl. Life Sciences, Vol. 45, S. 835 bis 842, 1989) untersucht; dabei konnten die vorstehend beschriebenen in vivo-Befunde bestätigt werden. Die Ergebnisse sind in den folgenden Tabellen für Kava-Extrakt (Tabelle 1), Methysticin (Tabelle 2) und Dihydromethysticin (Tabelle 3) festgehalten. Kava-Extrakt, Methysticin und Dihydromethysticin schützen die kultivierten Neuronen vor einer Schädigung durch eine Natriumcyanid-induzierte zytotoxische Hypoxie. Dabei wird der Proteingehalt der Kulturen als Indikator für Zellwachstum und Lebensfähigkeit der Zellen benutzt. Die angegebenen Proteingehalte sind Durchschnittswerte aus jeweils fünf bis sieben Tests (n gibt die Anzahl der durchgeführten Tests wieder).

Wie sich aus Tabelle 1 ergibt, fällt der Proteingehalt einer unbehandelten Vergleichskultur von 0,467 auf 0,317 mg/Flasche ab, wenn durch NaCN-Zusatz eine zytotoxische Hypoxie induziert wird. Wird die Kultur dagegen vor der Hypoxie-Induktion durch Zugabe von 1 mg/l Kava-Extrakt geschützt, dann fällt, verglichen mit der unbehandelten Vergleichskultur, der Proteingehalt nur auf 0,384 mg/Flasche ab. Relativ gesehen, steigt also der Proteingehalt der geschützten Kulturen von 0,317 auf 0,384 mg/Flasche signifikant an. Entsprechendes gilt für die neuroprotektive Wirksamkeit von Methysticin (vgl. Tabelle 2) und Dihydromethysticin (vgl. Tabelle 3).

**Tabelle 1**

| Der Einfluß des Kava-Extraktes auf neuronale Primärkulturen nach zytotoxischer Hypoxie mit NaCN. | | | |
|---|---|---|---|
| Kava-Extrakt (mg/l) | NaCN-Konzentration (mmol/l) | Proteingehalt (mg/Flasche) | n |
| 0 | 1 | 0,317 ± 0,022 | 7 |
| 1 | 1 | 0,384 ± 0,026 * * | 6 |
| 10 | 1 | 0,386 ± 0,043 * | 7 |
| 100 | 1 | 0,366 ± 0,021 * | 6 |
| 100 | 0 | 0,348 ± 0,018 + + | 6 |
| 0 | 0 | 0,467 ± 0,023 * * | 7 |

| | | | |
|---|---|---|---|
| Kruskal-Wallis H-Test: * p < 0,05; | | | |
| * * p < 0,01 (Vergleich mit Hypoxiekontrolle); | | | |
| + + p < 0,01 (Vergleich mit Normoxiekontrolle) | | | |

**Tabelle 2**

| Der Einfluß von Methysticin auf neuronale Primärkulturen nach zytotoxischer Hypoxie mit NaCN. | | | |
|---|---|---|---|
| Methysticin (µmol/l) | NaCN-Konzentration (mmol/l) | Proteingehalt (mg/Flasche) | n |
| 0 | 1 | 0,270 ± 0,014 | 7 |
| 0,01 | 1 | 0,290 ± 0,011 * | 7 |
| 0,1 | 1 | 0,294 ± 0,016 * | 7 |
| 1,0 | 1 | 0,290 ± 0,014 | 6 |
| 1,0 | 0 | 0,311 ± 0,009 * * | 6 |
| 0 | 0 | 0,345 ± 0,022 * * | 6 |

| | | | |
|---|---|---|---|
| Kruskal-Wallis H-Test: * p < 0,05; | | | |
| * * p < 0,01 (Vergleich zur Hypoxiekontrolle) | | | |

**Tabelle 3**

| Der Einfluß von Dihydromethysticin auf neuronale Primärkulturen nach zytotoxischer Hypoxie mit NaCN | | | |
|---|---|---|---|
| Dihydromethysticin (µmol/l) | NaCN-Konzentration (mmol/l) | Proteingehalt (mg/Flasche) | n |
| 0 | 1 | 0,535 ± 0,062 | 7 |
| 1 | 1 | 0,558 ± 0,051 | 6 |
| 10 | 1 | 0,627 ± 0,038 * | 6 |
| 100 | 1 | 0,597 ± 0,063 | 6 |
| 100 | 0 | 0,731 ± 0,050 * * | 6 |
| 0 | 0 | 0,706 ± 0,022 * | 5 |

| | | | |
|---|---|---|---|
| Kruskal-Wallis H-Test: * p < 0,05; | | | |
| * * p < 0,01 (Vergleich mit Hypoxiekontrolle) | | | |

## Patentansprüche

1. Verwendung eines Kava-Extraktes oder seiner Inhaltsstoffe als Neuroprotektivum.

2. Verwendung nach Anspruch 1 bei der Behandlung und Prophylaxe von Hirnleistungsstörungen.

3. Verwendung nach Anspruch 1 bei der Behandlung und Prophylaxe von Schlaganfällen.

4. Verwendung nach einem der Ansprüche 1 bis 3, dadurch gekennzeichnet, daß der Extrakt oder die Inhaltsstoffe in Form von Injektionslösungen, Infusionslösungen, Tabletten oder Tropfen, ggf. zusammen mit einem pharmazeutisch verträglichen Excipiens, vorliegen.
